# EUROPEAN PATENT SPECIFICATION

(11) **EP 0 573 492 B1**
(45) Date of publication and mention of the grant of the patent: **23.11.1994**
(21) Application number: 92905462.5
(22) Date of filing: 21.02.1992
(51) Int. Cl.: A61K 31/56

(54) **COMPOSITIONS FOR TOPICAL ADMINISTRATION CONTAINING FLUTICASONE PROPIONATE AND OXICONAZOLE OR ITS SALTS**
Pharmazeutische Zusammensetzungen enthaltend Fluticasonpropionat und Oxikonazol oder dessen Salz zur lokalen Anwendung
COMPOSITIONS A ADMINISTRATION LOCALE CONTENANT DU PROPRIONATE DE FLUTICASONE AINSI QUE DE L'OXICONAZOLE OU SES SELS

(30) Priority: 22.02.1991 GB 9103764
(43) Date of publication of application: 15.12.1993
(73) Proprietor: GLAXO GROUP LIMITED, Greenford, Middlesex UB6 0NN (GB)
(72) Inventor: HILL, Ernest, Arthur Glaxo Manufacturing, County Durham D112 8DT (GB)
(74) Representative: Brewer, Christopher Laurence, Dr.
(86) International application number: EP9200364
(87) International publication number: WO9214472

(56) References cited:
- Weekly Pharmacy Reports:"The Green Sheet", Vol. 40, No. 1, =7.01.91 "Glaxo's cultivate (fluticasone propionate).." p. 2,3.

## Description

The present invention relates to topical pharmaceutical compositions containing fluticasone propionate and oxiconazole as active ingredients.

Fluticasone propionate is the approved name for (S-fluoromethyl 6a,9a-difluoro-11b-hydroxy-16a-methyl-17a-propionyloxy-3-oxandrosta-1,4-diene 17b-carbothioate), which is disclosed in GB-A-2088877. Fluticasone propionate is a corticosteroid having good topical antiinflammatory activity with minimal liability to cause undesired systemic side effects.

Oxiconazole is the approved name for 1-(2-(4-chlorophenyl)-2-[(2,4-dichlorophenyl)methoxyimino)ethyl)-1H-imidazole. Oxiconazole has antifungal and antibacterial actions.

Topical corticosteroids are widely used for the treatment of inflammatory skin conditions such as, for example, inflammatory dermatoses and eczemas. There are many skin conditions such as, for example, infected dermatoses and eczema wherein infection by gram positive bacteria especially Staphylococci aureus and Streptococcus species and/or fungi, such as dermatophytes, yeasts and moulds, co-exist with inflammation.

We have now found that a combination of fluticasone propionate and oxiconazole is particularly effective in the treatment of skin disorders wherein inflammation and infection by bacteria and/or fungi coexist.

The invention thus provides fluticasone propionate and oxiconazole or a physiologically acceptable salt thereof, for use in combination in medicine.

It will be appreciated that reference to treatment is intended to include prophylaxis as well as the alleviation of established symptoms.

Suitable physiologically acceptable salts of oxiconazole include the hydrochloride, sulphate and nitrate salts. Preferably oxiconazole will be in the form of its nitrate.

According to a further aspect the invention provides topical pharmaceutical compositions comprising fluticasone propionate and oxiconazole or a physiologically acceptable salt thereof.

Compositions according to the invention can conveniently be formulated in conventional manner using one or more pharmaceutically acceptable carriers or excipients. Such compositions may take the form of, for example, ointments, lotions, creams, powders, drops (e.g. eye or ear drops) or sprays. Preferably the compositions of the invention will be in the form of creams or ointments.

Ointments may normally be prepared by melting white soft paraffin, (white petrolatum) incorporating any additives e.g. surfactants and solvents, and blending in a slurry of the drug in a minimum quantity of liquid paraffin. The melt is then cooled under controlled conditions and stirred until solidification occurs.

Creams may normally be prepared by combining the oily phase of an ointment as a melt as described above, with suitable oil and water soluble surfactants and an aqueous phase containing the drug and suitable anti-microbial preservatives, homogenising to form the cream and stirring gently until cool.

Compositions according to the invention will generally contain additional excipients, for example preservatives (such as benzoic acid), emulsifying agents (such as polysorbates, e.g. polysorbate 60) and viscosity enhancing agents (such as cetostearyl alcohol).

It is an advantage of the compositions of the present invention that, due to their improved effectiveness, they generally need be applied only once or twice daily. This is in contrast to known combined therapies comprising a corticosteroid and an antibacterial and/or antifungal agent, which all require multiple daily applications.

The amount of fluticasone propionate contained in the compositions of the invention will depend on the particular type of formulation concerned but will generally be in the range of from 0.0005% to 1.0% by weight of the formulation, preferably about 0.05% w/w.

The amount of oxiconazole in the compositions will also depend on the particular formulation but the content of free base will generally be in the range of from 0.01 to 10.00% by weight of the formulation, preferably about 1.00% w/w.

The invention is further illustrated by the following non-limiting example :

| Example 1 | |
|---|---|
| | % w/w |
| Oxiconazole Nitrate | 1.147* |
| Fluticasone Propionate (micronised) | 0.05 |
| Cetostearyl Alcohol | 10.00 |
| White Soft Paraffin | 10.00 |
| Polysorbate 60 | 2.50 |
| Propylene Glycol | 10.00 |
| Benzoic Acid | 0.20 |
| Purified Water | to 100.00 |

| | |
|---|---|
| * equivalent to 1.00% oxiconazole base. | |

## Claims

1. Fluticasone propionate and oxiconazole or a pharmaceutically acceptable salt thereof, for use in combination in medicine.

2. Fluticasone propionate and oxiconazole or a pharmaceutically acceptable salt thereof for use in combination in medicine according to Claim 1 wherein the oxiconazole is present as oxiconazole nitrate.

3. Fluticasone propionate and oxiconazole or a pharmaceutically acceptable salt thereof for use in combination according to Claim 1 or Claim 2 wherein the ratio of fluticasone propionate to oxiconazole is about 0.05:1.00% w/w.

4. Use of fluticasone propionate and oxiconazole or a pharmaceutically acceptable salt thereof for the manufacture of a medicament for the treatment of skin disorders.

5. A pharmaceutical formulation adapted for topical administration and comprising fluticasone propionate, oxiconazole or a pharmaceutically acceptable salt thereof and pharmaceutically acceptable carrier therefor.

6. A pharmaceutical formulation according to Claim 6 wherein oxiconazole is present as oxiconazole nitrate.

7. A pharmaceutical formulation according to Claim 6 or Claim 7 in the form of an ointment, lotion, cream, powder, drops or sprays.

8. A pharmaceutical formulation as claimed in any one of Claims 6 to 8 wherein the fluticasone propionate is present in an amount of from 0.0005% to 1% by weight and the oxiconazole is present in an amount of 0.01 to 10.00% by weight.

9. A pharmaceutical formulation as claimed in any one of Claims 6 to 9 wherein the fluticasone propionate is present in an amount of about 0.05% w/w and the oxiconazole is present in a amount of about 1.00% w/w based upon the free base.

10. A method for the preparation of a pharmaceutical formulation adapted for topical administration and comprising fluticasone propionate and oxiconazole or a pharmaceutically acceptable salt thereof as active ingredients together with a pharmaceutically acceptable carrier therefor which method comprises admixture of the active ingredients and the carrier.

## Patentansprüche

1. Fluticasonpropionat und Oxiconazol oder ein pharmazeutisch annehmbares Salz davon zur Verwendung in Kombination in der Medizin.

2. Fluticasonpropionat und Oxiconazol oder ein pharmazeutisch annehmbares Salz davon zur Verwendung in Kombination in der Medizin nach Anspruch 1, wobei das Oxiconazol als Oxiconazolnitrat vorliegt.

3. Fluticasonpropionat und Oxiconazol oder ein pharmazeutisch annehmbares Salz davon zur Verwendung in Kombination nach Anspruch 1 oder 2, wobei das Gewichtsverhältnis von Fluticasonpropionat zu Oxiconazol etwa 0,05:1,00% Gew./Gew. ist.

4. Verwendung von Fluticasonpropionat und Oxiconazol oder eines pharmazeutisch annehmbaren Salzes davon zur Herstellung eines Arneimittels zur Behandlung von Störungen der Haut.

5. Pharmazeutisches Präparat zur topischen Verabreichung, dadurch **gekennzeichnet,** daß es Fluticasonpropionat, Oxiconazol oder ein pharmazeutisch annehmbares Salz davon und einen pharmazeutisch annehmbaren Träger hierfür enthält.

6. Pharmazeutisches Präparat nach Anspruch 5, dadurch **gekennzeichnet,** daß Oxiconazol als Oxiconazolnitrat vorliegt.

7. Pharmazeutisches Präparat nach Anspruch 5 oder 6, dadurch **gekennzeichnet,** daß es in Form einer Salbe, einer Lotion, einer Creme, eines Pulvers, von Tropfen oder von Sprays vorliegt.

8. Pharmazeutisches Präparat nach einem der Ansprüche 6 bis 7, dadurch **gekennzeichnet,** daß das Fluticasonpropionat in einer Menge von 0,0005 bis 1 Gew.-% und das Oxiconazol in einer Menge von 0,01 bis 10,00 Gew.-% vorliegen.

9. Pharmazeutisches Präparat nach einem der Ansprüche 6 bis 8, dadurch **gekennzeichnet,** daß das Fluticasonpropionat in einer Menge von etwa 0,05% Gew./Gew. und das Oxiconazol in einer Menge von etwa 1,00% Gew./Gew., bezogen auf die freie Base, vorliegt.

10. Verfahren zur Herstellung eines pharmazeutischen Präparats zur topischen Verabreichung, das Fluticasonpropionat und Oxiconazol oder ein pharmazeutisch annehmbares Salz davon als Wirkstoffe zusammen mit einem pharmazeutisch annehmbaren Träger hierfür enthält, dadurch **gekennzeichnet,** daß man die Wirkstoffe und den Träger miteinander vermischt.

## Revendications

1. Propionate de fluticasone et oxiconazole ou un sel pharmaceutiquement acceptable de celui-ci, destinés à l'utilisation en combinaison en médecine.

2. Propionate de fluticasone et oxiconazole ou un sel pharmaceutiquement acceptable de celui-ci, pour l'utilisation en combinaison en médecine selon la revendication 1, caractérisés en ce que l'oxiconazole est présent sous forme de nitrate d'oxiconazole.

3. Propionate de fluticasone et oxiconazole ou un sel pharmaceutiquement acceptable de celui-ci, pour l'utilisation en combinaison en médecine selon la revendication 2, caractérisés en ce que le rapport du propionate de fluticasone à l'oxiconazole est d'environ 0,05:1,00% p/p.

4. Utilisation du propionate de fluticasone et de l'oxiconazole ou d'un sel pharmaceutiquement acceptable de celui-ci, en vue de la fabrication d'un médicament destiné au traitement de troubles ou désordres cutanés.

5. Composition pharmaceutique adaptée à l'administration topique et comprenant du propionate de fluticasone, de l'oxiconazole ou un sel pharmaceutiquement acceptable de celui-ci, ainsi qu'un véhicule ou excipient pharmaceutiquement acceptable pour ceux-ci.

6. Composition pharmaceutique suivant la revendication 5, caractérisée en ce que l'oxiconazole est présent sous forme de nitrate d'oxiconazole.

7. Composition pharmaceutique suivant la revendication 5 ou la revendication 6, caractérisée en ce qu'elle se présente sous forme d'onguents ou pommades, de lotions, de crèmes, de poudres, de gouttes ou de sprays.

8. Composition pharmaceutique suivant l'une quelconque des revendications 5 à 7, caractérisée en ce que le propionate de fluticasone est présent en une proportion de 0,0005% à 1% en poids et l'oxiconazole est présent en une proportion de 0,01 à 10,00% en poids.

9. Composition pharmaceutique suivant l'une quelconque des revendications 5 à 8, caractérisée en ce que le propionate de fluticasone est présent en une proportion d'environ 0,05% p/p et l'oxiconazole est présent en une proportion d'environ 1,00% p/p, sur base de la base libre.

10. Procédé de préparation d'une composition pharmaceutique adaptée à l'administration topique et comprenant du propionate de fluticasone et de l'oxiconazole ou un sel pharmaceutiquement acceptable de celui-ci, à titre d'ingrédients actifs, en même temps qu'un excipient ou véhicule pharmaceutiquement acceptable pour ceux-ci, caractérisé en ce que l'on mélange les ingrédients actifs et le véhicule ou excipient.
